# EUROPEAN PATENT SPECIFICATION

(11) **EP 0 473 645 B1**
(45) Date of publication and mention of the grant of the patent: **18.03.1998**
(21) Application number: 90907700.0
(22) Date of filing: 22.05.1990
(51) Int. Cl.: A01N 25/04, A01N 25/24, A01N 25/26, A61K 7/00, A61K 7/40, A61K 9/127

(54) **INSECT REPELLANT**
ABSTOSSMITTEL FÜR INSEKTEN
AGENT DE REPULSION D'INSECTES

(30) Priority: 25.05.1989 US 356506
(43) Date of publication of application: 11.03.1992
(73) Proprietor: TECHNOLOGY UNLIMITED INCORPORATED, Wooster, OH 44691 (US)
(72) Inventor: GEHO, W., Blair, Wooster, OH 44691 (US); LAU, John, R., Wooster, OH 44691 (US); WOODS, Darryl, H., Dalton, OH 44691 (US)
(74) Representative: Jones, Stephen Anthony
(86) International application number: US9002725
(87) International publication number: WO9014096

(56) References cited:
- WO-A-85/00011
- WO-A-85/00515
- GB-A- 1 167 369
- US-A- 4 241 046
- US-A- 4 477 467
- US-A- 4 515 736
- US-A- 4 596 864
- US-A- 4 666 747
- US-A- 4 708 861
- US-A- 4 731 210
- US-A- 4 839 175
- US-A- 4 855 090
- Drug Carrier Systems, 1989, (JULIANO, R.L.) "Liposomes as Drug Carriers in the Therapy of Infectious Diseases.

## Description

### Field of the Invention

Emulsions are formed from molecules that are not very soluble in aqueous media, but are soluble in organic solvents.

A species of the field is the establishment of a delivery system using a liposomal emulsion delivery system for targeting and anchoring aromatic hydrocarbons, such as mosquito repellants and insecticides, to the stratum corneum.

Liposome and vesicle will be used interchangeably herein, because the art is not sufficiently exact with respect to the parameters of nomenclature.

### Description of Prior Art

Liposomes are known to encapsulate and thereby sequester water soluble pharmacological agents. Thus, these agents are separated from the surrounding media. The liposomes have potential for cite specific delivery, as presented by the prior art, for their use as carriers to enhance therapeutic and protective indices.

Achieving controlled release of volatile substances such as insect repellants, has been the object of intense effort for several decades. However, only a few reports of actual success to limited systems are known. Literature studies indicate that most success in this area has been through the use of polymers, resins and impregnated gel beads and silica. These prior art systems provide only minimal accuracy and afford less than satisfactory control over release of the product.

This invention distinguishes over all known prior art, including intensive literature studies, by the discovery that formation of bipolar lipids into vesicles in the presence of organic molecules that are minimally soluble in aqueous media, exhibit a phenomena of physical entrapment of the organic solvent in the manner illustrated in the drawings. The organic solvent would not ordinarily be expected to fill the core of a vesicle/liposome during formation, because the outermost portion of a lipid membrane, both on the exterior surface and on the core volume wall, is a hydrophilic sphere and therefore it is generally assumed that an organic solvent would not be encapsulated.

It is an object of this invention to provide a slow release delivery system for an insect repellent or insecticide which is soluble in an organic solvent, by sequestering the chemical in a bipolar vesicle, and to provide a target molecule for such sequestered agent, the target molecule being selected to have affinity for stratum corneum.

WO 88/06881 discloses liposomes incorporating lipophilic substances, including insecticides and insect repellant agents, and also liposomes incorporating targeting molecules (eg monoclonal antibodies, lectins, peptide hormones, glycolipids, galactocerebrosides, etc). The targeting molecules are said to direct the liposomes to particular targets in order to allow release of encapsulated material at a specific biological location.

US 4,241,046 discloses liposomes comprising bioactive substances, including insecticides. Exemplified are liposomes encapsulating peptides, nucleic acids and bacteria.

US 4,666,747 discloses liposomes incorporating insecticides. It is stated that the adhesiveness of such liposomes may be enhanced by changing the electrostatic properties of the liposome surface, eg by using acidic phospholipids or long chain fatty acids in the liposome structure.

### SUMMARY OF THE INVENTION

The term "burden" as used herein refers in general to an insecticidal or insect repellant chemical for application to a living host skin surface, to elicit a desired response.

The burden is sequestered in a bipolar lipid vesicle, or entrapped in a fragment thereof.

Application of an environment enhancement chemical to the skin of a warm blooded host is accomplished by per-forming liposomal emulsification of the enhancement chemical, and anchoring the liposome to the stratum corneum by means of sodium or zinc pyridinethione as targeting molecule having a lipophilic moiety engaged into the liposome, and having a moiety with affinity for the stratum corneum.

A specific example is the emulsification of molecules that are not very soluble in aqueous media but show solubility for lipid phase and organic solvents. For example, one embodiment is 6,12 (2 Ethyl-1, 3,-hexanediol) and N,N-Diethyl-m-toluamide (DEET).

Flea and tick repellants and/or insecticides are further examples.

The drawings illustrating this invention show the discovered unique sequestering of the described class of water insoluble burden. Liposomes cannot be formed in an organic solvent, and water insoluble chemicals cannot be trapped in a core volume of a lipid vesicle. This invention discloses the phenomena of physically sequestering organic soluble material.

Then, by attaching a targeting molecule an article of manufacture is produced which will attach to a targeted skin cell. For example, by targeting the human stratum corneum with pyridinethione, sequestered DEET will gradually escape its sequestering lipid, and repel or kill insects for an extended period of time.

### BRIEF DESCRIPTION OF THE DRAWINGS

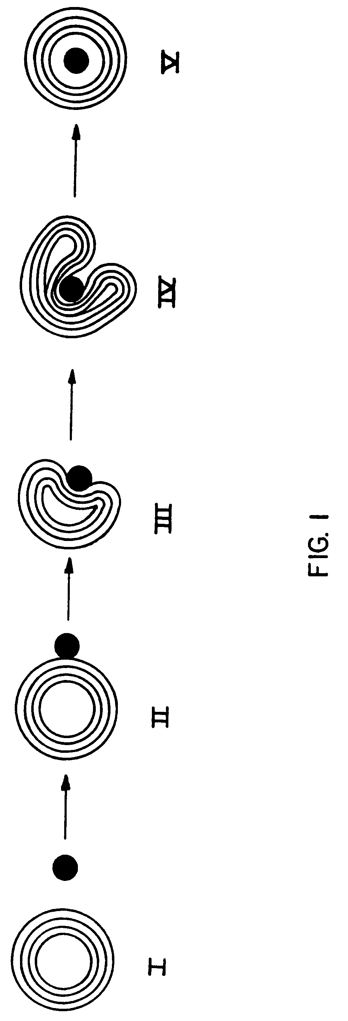

The single figure is a five step sequence taken from electron micrographs.

### DEFINITION

Because the invention is in the unique means for dispensing an insect repellant or insecticide, and not in the substance per se, reference hereinafter for disclosure will be limited to "insect repellant" for economy of expression.

### DETAILED DESCRIPTION

### First Example

One preferred embodiment of the invention is to provide an article of manufacture for repelling or killing insects, comprising a substantially water insoluble organic compound DEET, or a pesticide, loaded to a bipolar lipid body. A targeting molecule, for anchoring the lipid body, has a lipid soluble moiety united to the lipid body, and a target moiety having affinity for stratum corneum.

As stated, the prior art method of making vesicles or liposomes is to subject a mixture of bipolar lipid, such as egg lecithin, and an aqueous solution of the intended burden, to high energy input such as sonication or microfluidization. The bipolar lipid breaks into smaller units and forms bladders known as a vesicle or liposome. In the process some of the aqueous media is captured in the core volume of the liposomes. This basic known system is satisfactory for aqueous media but is inoperative for organic molecules that are minimally soluble in aqueous media.

In this example, a lecithin is first polytroned and then microfluidized or sonicated to create very large multilamellar liposomes. Although a separate step may be employed, it has been found practical to carry out a simultaneous reaction by including in the mixture of lecithin and aqueous media, a burden such as DEET. The DEET molecule possesses hydrophobic (lipid seeking) groups such as the ethyl moiety and the paramethylbenzene rings. The lipid seeking nature of the DEET molecule allows it to seek and penetrate a large multilamellar liposome so that the lipid domain of the liposome actually is disrupted and penetrated by the DEET molecule. The capture of the DEET molecule is illustrated in the five-step illustration in the drawing, which are reproductions of actual images from electron miscroscopy. The small black spot represents a particle of DEET and the multilamellar rings represent a larger liposome.

The liposome, no longer being intact, is simply a large multilamellar array of lipid which seeks to repair itself thermodynamically and in so doing emulsifies the DEET particle or molecule by surrounding the DEET with the large multilamellar array. The DEET particle thus has been sequestered and protected by lipid. This is not a liposome or a vesicle in the classical sense because the core volume is not aqueous but rather is organic. The organic particle of DEET seeks to exclude core volume water.

This concept may be considered as a semi-classical emulsion. It has all the characteristics of an emulsion. It breaks upon freezing and warming causing the DEET to coalesce and partition into a larger separate phase. The liposomal lamellae are acting as an emulsifying agent. The major portion of the lamellae evidence a distorted non-spherical pattern, as illustrated in the drawing, as they attempt to emulsify the DEET particles progresses.

The manner in which the emulsification effect differs from classical emulsions is that the emulsifying agent has lamellae or onion skin kinds of structures and these structures have been preformed in a single step. A multiple protective layer pattern has been developed that will emulsify the DEET.

The described liposomal emulsion system has captured or sequestered DEET in a new way based on emulsion chemistry. It is apparent that once the DEET has been emulsified by the onion skin lamellae, its escape to the atmosphere is restricted and slowed. This concept forms the basis for timed release of water insoluble substances such as DEET and prolongs their duration of action.

The above-described article of manufacture may be employed in the described state and can cling to skin and hair for a period of time. However, a vastly improved result is obtained by employing a variety of skin substantive molecules such as pyridinethione and the derivatives described herein to anchor the lipid and its burden to skin of the host for the purpose of maintaining a slow time-release of the active product onto the skin surface and surrounding area.

The target molecule pyridinethione is a molecule having a lipid soluble moiety and a hydrophilic moiety and therefore the lipid soluble moiety will penetrate the wall of the emulsified array and extend the hydrophilic moiety away from the surface to act as a targeting agent for attaching the completed article of manufacture to the corneum stratum.

### Second Example

Close examination of the mixture which has been polytroned and subjected to either sonication or microfluidization reveals that not all of the lipid material has been formed into perfect liposomes as illustrated in the drawing. There remains fragments of the liposomes which have a slightly curved configuration, as well as sheet-like fragments of bipolar lipid. If the intensity of the sonication or microfluidization is off of ideal, the resultant product can be essentially all fragments or bits of sheet bipolar lipid with few well formed liposomes. It is this type of product which heretofore has been considered to be essentially useless because of the small amount of well formed liposomes. It has been discovered, in working with this material, however, that contrary to expectations, a very effective delivery of DEET to the stratum corneum has actually been achieved.

The explanation for this unexpected phenomena is known as partitioning within multilamellar bilayers. It is a known fact that highly immiscible fluids, such as oil and water, do have some degree of solubility one in the other. The amount of solubility will vary according to the particular ingredients, which solubility is obtainable from standard chemical tables.

Therefore, it has been discovered that those fragments and sheets which are not sufficiently large to envelope the DEET as illustrated, for example by the completely formed vesicles, will nevertheless carry some of the DEET and also will accept the targeting molecule. Therefore, the degree of perfection toward forming of perfect vesicles is greatly reduced and the efficiency of the manufacturing process greatly increased.

### Targeting Procedure

Applicants recognize the ability of chelated zinc pyridinethione (ZPT) to adhere to stratum corneum. ZPT has the following structural formula:

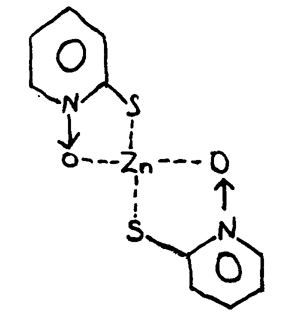

Since the zinc complex of ZPT shown above is much less soluble in aqueous media because of the ligand formation by the ionizable and hydrophilic functional groups, such as the sulfhydrals and oxygens, this molecule is an appropriate candidate as a liposome targeting agent. It can be incorporated in a one-step procedure into the liposome membrane during the time of sonication and microfluidization.

The procedure used for making the product as tested is set forth below:
1. 120 mg of the sodium salt of pyridinethione is dissolved in 525 ml of deionized water and allowed to stir at ambient temperature for 15 minutes.
2. 120 g of hydroxylated lecithin is weighed into an 800 ml beaker while 525 ml of solubilized sodium pyridinethione is added in 100 ml aliquots. During pyridinethione addition, the sample is slowly polytroned with an ultra-Turrax TP-18/1051 polytron from Janke and Kunkel IKA Laboratory, at setting #4 for 15 minutes.
3. After the lecithin had been mixed, 180 ml of 100% diethyl-m-toluamide (DEET) was added to the 600 ml of sodium pyridinethione lecithin solution in three aliquots of 60 ml each, five minutes apart. After the last aliquot, the total volume of 825 ml was allowed to mix while polytroning on setting #4 for 10 minutes. Then, 495 ml of deionized water was added and allowed to mix while polytroning on setting #4 for 30 minutes. The total volume of the solution at this point was 1320 ml.
4. The 1,320 ml of the pyridinethione-lecithin DEET suspension was processed through the microfluidizer at a head pressure of 20 psig and a shear pressure of 2,500 psig. The total recoverable volume of 1,320 ml was recycled three times through the microfluidizer under the above conditions. The initial starting temperature of the suspension was 20 degrees C, and the final temperature following microfluidization was 63 degrees C.

## Claims

1. An insect repellent or insecticidal composition for application to the skin of a user, which composition comprises an insect repellant or insecticide soluble in organic solvents and substantially insoluble in water, the insect repellant or insecticide being encapsulated within liposomes dispersed in an aqueous environment, characterised in that said liposomes further comprise sodium pyridinethione or zinc pyridinethione as an anchoring molecule attached to the liposomes so as to confer on the liposomes an affinity for stratum corneum.

2. A composition as claimed in Claim 1, wherein the composition contains sodium pyridinethione.

3. A composition as claimed in Claim 1, wherein the composition contains zinc pyridinethione.

4. A composition as claimed in Claim 1 or Claim 2, comprising N,N-diethyl-m-toluamide.

5. A composition as claimed in any preceding claim, wherein the liposomes comprise hydroxylated lecithin.

6. A process for the manufacture of a composition as claimed in Claim 5, which process comprises the steps of
a) forming a dispersion of hydroxylated lecithin in water,
b) forming a solution of sodium pyridinethione or zinc pyridinethione in water,
c) sonicating the dispersion formed in step a) whilst adding the solution formed in step b), to form a liposomal dispersion, and
d) mixing said liposomal dispersion with an insect repellant or insecticide soluble in organic solvents and substantially insoluble in water.

## Patentansprüche

1. Insektenabwehrmittel oder Insekten vernichtende Zusammensetzung zum Auftragen auf der Haut des Anwenders, wobei die Zusammensetzung ein in organischen Lösungsmitteln lösliches und im wesentlichen wasserunlösliches Insektenabwehrmittel oder Insektizid enthält, wobei das Insektenabwehrmittel oder Insektizid in in einem wäßrigen Medium dispergierten Liposomen eingekapselt ist, dadurch gekennzeichnet, daß die Liposome des weiteren Natriumpyrithion oder Zinkpyrithion als an die Liposome anhaftendes Ankermolekül erthalten, um den Liposomen dadurch eine Affinität zur Hornschicht des Nagels zu verleihen.

2. Zusammensetzung nach Anspruch 1, wobei die Zusammensetzung Natriumpyrithion enthält.

3. Zusammensetzung nach Anspruch 1, wobei die Zusammensetzung Zinkpyrithion enthält.

4. Zusammensetzung nach Anspruch 1 oder 2, die N,N'-Diethyl-m-toluamid enthält.

5. Zusammensetzung nach einem der vorhergehenden Ansprüche, in der die Liposome hydroxyliertes Lecithin enthalten.

6. Verfahren zur Herstellung einer Zusammensetzung nach Anspruch 5, welches folgende Schritte umfaßt:
a) Bildung einer Dispersion von hydroxyliertem Lecithin in Wasser,
b) Bildung einer Lösung von Natriumpyrithion oder Zinkpyrithion in Wasser
c) Beschallung der in Schritt a) gebildeten Dispersion unter Zusatz der in Schritt b) gebildeten Lösung zur Bildung einer Liposomendispersion und
d) Mischen der Liposomendispersion mit einem in organischen Lösungsmitteln löslichen und im wesentlichen wasserunlöslichen Insektenabwehrmittel oder Insektizid.

## Revendications

1. Insectifuge ou composition insecticide destinée à une application sur la peau d'un utilisateur, ladite composition comprenant un insectifuge ou un insecticide soluble dans des solvants organiques et essentiellement insoluble dans l'eau, l'insectifuge ou l'insecticide étant encapsulé dans des liposomes dispersés dans un environnement aqueux, caractérisé en ce que lesdits liposomes comprennent en outre de la pyridinethione de sodium ou de la pyridinethione de zinc à titre de molécule d'ancrage fixée aux liposomes de façon à conférer aux liposomes une affinité pour la couche cornée.

2. Composition selon la revendication 1, dans laquelle la composition contient de la pyridinethione de sodium.

3. Composition selon la revendication 1, dans laquelle la composition contient de la pyridinethione de zinc.

4. Composition selon l'une quelconque des revendications 1 ou 2, comprenant du N,N-diéthyl-m-toluamide.

5. Composition selon l'une quelconque des revendications précédentes, dans laquelle les liposomes comprennent de la lécithine hydroxylée.

6. Procédé pour la préparation d'une composition selon la revendication 5, ledit procédé comprenant les étapes consistant à:
a) former une dispersion de lécithine hydroxylée dans de l'eau,
b) former une solution de pyridinethione de sodium ou de pyridinethione de zinc dans de l'eau,
c) soumettre à un traitement aux ultrasons la dispersion obtenue à l'étape a) tout en ajoutant la solution formée à l'étape b) pour former une dispersion liposomique, et
d) mélanger ladite dispersion liposomique avec un insectifuge ou un insecticide soluble dans des solvants organiques et essentiellement insoluble dans l'eau.
